# FASCICULE DE BREVET EUROPEEN

(11) **EP 3 215 171 B1**
(45) Date de publication et mention de la délivrance du brevet: **27.01.2021**
(21) Numéro de dépôt: 15798535.9
(22) Date de dépôt: 03.11.2015
(51) Int. Cl.: A61K 35/20, A61K 38/01, A23L 33/17, A23C 21/06, A23J 3/10, A23L 33/00, A61P 3/00, A23C 9/142, A23L 33/185, A23L 33/19, A61K 36/48

(54) **COMPOSITION DE NUTRITION ENTERALE**
ENTERALE ERNÄHRUNGSZUSAMMENSETZUNG
ENTERAL NUTRITION COMPOSITION

(30) Priorité: 03.11.2014 FR 1460583; 03.12.2014 FR 1461860
(43) Date de publication de la demande: 13.09.2017
(73) Titulaire: Even Santé Industrie, 29260 Ploudaniel (FR)
(72) Inventeur: RANC, Anne, 29440 Treflaouenan (FR); LE PALUD, Jean-Marc, 29860 Plabennec (FR); FOSSEUX, Anne, 29200 Brest (FR)
(74) Mandataire: IP Trust
(86) Numéro de dépôt international: PCT/FR2015/052955
(87) Numéro de publication internationale: WO 2016/071621

(56) Documents cités:
- EP-A1- 0 686 396
- WO-A1-2009/072886
- WO-A1-2009/113845
- WO-A1-2011/152726
- WO-A1-2013/129912
- US-A1- 2012 142 587

## Description

### Domaine de l'invention

L'invention porte sur une composition de nutrition entérale pour personnes âgées, malades, affaiblies ou en état de dénutrition liée à la maladie. La présente invention est à destination des adultes.

Les compositions de nutrition entérale sont en général des aliments stérilisés.

L'invention s'inscrit dans le domaine des compositions de nutrition entérale stables, utilisables en source principale d'alimentation ou en complément. L'invention s'inscrit plus précisément dans le domaine des compositions nutritionnelles entérales liquides, hyperprotéinées, hyperénergétiques, unidoses et prêtes à l'emploi.

### Contexte général de l'invention

Le corps humain est un organisme complexe. Le maintien des fonctions vitales nécessite de l'énergie qui est apportée par l'alimentation.

L'alimentation apporte de l'énergie quantifiable sous forme de kilojoules ou de kilocalories.

Le bon fonctionnement du corps nécessite une alimentation suffisante et équilibrée. L'énergie doit être apportée par des protéines, des sucres et des matières grasses. Les protéines sont indispensables à l'activité musculaire, les matières grasses permettent de stocker de l'énergie libérable en fonction des besoins du corps, et les sucres apportent une source immédiatement disponible d'énergie. Ces sources caloriques sont donc complémentaires. Elles doivent être complétées par des apports en vitamines et minéraux qui interviennent dans l'équilibre physiologique des différentes fonctions vitales du corps.

Cet équilibre peut être rompu en cas de malnutrition due par exemple à un trouble de l'alimentation (anorexie...), ou de dénutrition conséquente à une situation pathologique ou chez les personnes âgées qui ne s'alimentent plus correctement de façon autonome.

Les besoins nutritionnels évoluent au cours de la vie. Par exemple, les personnes âgées ont des besoins réduits en lipides, dus à une diminution d'activité et à un ralentissement du renouvellement cellulaire.

Certaines personnes, par exemple à cause de la maladie, ne sont plus capables de s'alimenter correctement, voire même de s'alimenter complètement. Il est donc nécessaire de mettre en place une alimentation de supplémentation ou de substitution afin de subvenir à leurs besoins nutritionnels.

Lorsque le tube digestif est toujours fonctionnel, la nutrition entérale est privilégiée. Elle peut se faire avec ou sans sonde avec des compositions nutritionnelles adaptées en terme de profil nutritionnel, de viscosité ou encore de volume.

A titre d'exemple, certains patients ne peuvent pas ingérer de volumes importants d'aliments. Il s'agit par exemple des patients cachectiques en lien ou non avec une pathologie, par exemple le syndrome d'immunodéficience acquise, un cancer, des maladies respiratoires ou infectieuses, ou des traumatismes.

### Etat de la technique

L'on connaît de l'état de la technique des compositions de nutrition entérale liquide.

La demande de brevet WO2009/072885 se rapporte à des compositions nutritionnelles entérales liquides riches en énergie et en protéines qui contiennent du caséinate et de la caséine micellaire, et en option une faible quantité de lactosérum.

La demande de brevet WO2009/072884 porte sur une composition liquide de nutrition entérale comprenant des protéines intactes à faible volume permettant la préparation d'aliments avec une forte teneur protéique et énergétique. Ces protéines consistent en des caséines micellaires, des caséinates, du petit lait et des protéines de soja ou de pois.

La demande de brevet WO02/098242 décrit un supplément oral liquide à haute densité énergétique (2,25 kcal/ml) dont la source protéique est constituée de caséinates et de protéines de soja.

La demande US2012/0142587 décrit une composition nutritionnelle entérale liquide comprenant une quantité élevée de protéines apportée principalement par des caséines micellaires et dans laquelle la quantité totale d'ions métalliques monovalents est inférieur à 25mg par gramme de protéine.

La demande WO2013/129912 décrit une composition nutritionnelle liquide comprenant entre 6 et 20 g de protéines pour 100ml et dans laquelle la fraction protéique comprend au moins des caséines micellaires.

La demande WO2011/152726 décrit une composition nutritionnelle dense en nutriments présentant une viscosité stable et une longue conservation grâce à l'utilisation de fibres diététiques acides ou anioniques.

La demande WO2009/072886 décrit une composition nutritionnelle entérale liquide comprenant une quantité élevée de protéines comprise entre 10 et 20 g par 100 ml de composition, les protéines étant principalement apportées par des caséines micellaires et fournissant entre 10 et 100% de l'énergie totale de la composition.

La demande WO2009/113845 décrit une composition nutritionnelle entérale liquide comprenant 9 à 20g de protéines globulaires non-hydrolysées comme celles du petit-lait, du soja ou de pois par 100 ml de composition.

### Inconvénients de l'art antérieur

Les solutions de l'art antérieur ne permettent pas d'avoir une source protéique homogène et facile d'utilisation. Les protéines de lait utilisées dans les solutions de l'art antérieur ne respectent pas la composition naturelle du lait en terme de caséines micellaires et de petit-lait. En effet, les caséinates ne sont pas présents en tant que tels dans le lait.

Les caséinates, notamment les caséinates de sodium et de potassium, sont connus pour augmenter la viscosité des compositions dans lesquels ils sont utilisés comme source protéique. Ainsi une composition comprenant des caséinates a une viscosité plus forte qu'une composition comprenant des caséines micellaires. Les caséinates de calcium sont également particulièrement responsables de la formation de cristaux de calcium qui impactent négativement la stabilité de la composition de nutrition entérale, réduisant les dates limites d'utilisation optimale (DLUO). Les caséinates de calcium quant à eux ne sont pas ou peu solubles et ont tendance à précipiter sous l'effet de l'acidité. Ceci est particulièrement problématique pour la stabilité des compositions qui ont tendance à s'acidifier en vieillissant.

### Solution apportée par l'invention

La présente invention se propose de pallier aux inconvénients de l'art antérieur en portant sur une composition de nutrition entérale complète thermostabilisée à longue conservation comportant 8 à 12 g de protéines par 100 mL de composition, lesdites protéines apportant environ 16% de la teneur énergétique totale, lesdites protéines consistant en un mélange de protéines totales de lait et de protéines végétales, lesdites protéines totales de lait consistant en des caséines micellaires et du petit-lait, lesdites caséines micellaires représentant au moins 70% en poids des protéines totales, ladite composition ayant une densité énergétique d'au moins 8,4 kJ/mL, soit 2,0 kcal/ml, et une viscosité comprise entre 100 et 300 mPa.s mesurée avec un viscosimètre rotationnel à 20°C à 50 s⁻¹.

La demanderesse a constaté avec étonnement que la composition selon l'invention présentait une stabilité importante permettant une DLUO de plusieurs mois, avantageusement de 12 mois, tout en ayant une viscosité suffisante pour être adaptée à une nutrition entérale de patients dénutris, malades, âgés ou présentant des carences. La demanderesse a constaté de façon étonnante que la viscosité de la composition était suffisante et ce même en l'absence de caséinates. La composition selon l'invention conserve également de bonnes propriétés organoleptiques contrairement à des compositions nutritionnelles à base de protéines non natives comme des peptides ou des acides aminés libres. La préservation des propriétés organoleptiques est primordiale pour maintenir l'envie de se nourrir des personnes dénutries, malades, en convalescence ou âgées.

La composition de nutrition entérale est liquide et buvable, sa viscosité étant comprise 100 et 300 mPa.s mesurée avec un viscosimètre rotationnel à 20°C à 50 s⁻¹. Le viscosimètre rotatif est de préférence un viscosimètre de type cup and bob, par exemple un DIN coaxial cylinder.

L'on comprend que la composition selon l'invention ne contient pas de caséinates comme source protéique. La source protéique est constituée de caséines micellaires, de petit-lait et de protéines végétales. La source protéique est diversifiée entre protéines animales et protéines végétales. Les caséines micellaires sont comprises comme des caséines micellaires natives, c'est à dire des caséines sous forme de micelles. La source protéique est comprise comme les ingrédients susceptibles d'apporter des caséines micellaires, du petit-lait et des protéines végétales.

La composition selon l'invention est destinée à l'alimentation d'adultes. Elle présente une source protéique améliorée.

La composition selon la présente invention présente un profil en acides aminés particulièrement adapté aux besoins nutritionnels des personnes dénutries, en convalescence, âgées ou malades chroniques. Les compositions apportent notamment les acides aminés essentiels que l'organisme humain n'est pas capable de synthétiser. L'indice chimique, calculé selon la méthode FAO, des compositions selon l'invention est proche de 100, voire supérieur à 100, ce qui est la valeur idéale, alors que les compositions de l'état de l'art comprenant des caséinates ont un indice chimique inférieur à 100. La digestibilité des compositions selon l'invention est bonne.

L'on comprend par « complète » une composition de nutrition apportant tous les éléments nutritionnels nécessaires.

La composition selon l'invention est prête à l'emploi. La composition est conditionnée en bouteille, pack, briquette ou tout autre emballage. La composition est conditionnée en format unidose, généralement de 50, 100, 125, 150, 200, 250 ou 300 mL, préférentiellement de 125 mL. La composition ainsi conditionnée est de longue conservation, avec une DLUO d'au moins 6 mois, préférentiellement d'au moins 9 mois, avantageusement d'au moins 12 mois.

Il est connu de l'Homme du métier que les caséines micellaires peuvent précipiter selon l'environnement ionique et le pH, ce qui diminue la stabilité de la composition. La demanderesse a constaté que ce problème était résolu en utilisant une source protéique multiple conformément à l'invention, en adaptant les minéraux présents dans la composition, ainsi qu'en contrôlant le pH. Le pool protéique selon l'invention résiste particulièrement bien à la stérilisation lors du procédé de préparation de la composition. Il est ici compris par pool protéique les différentes sources de protéines mises en œuvre en tant qu'ingrédients dans la fabrication.

Les compositions de nutrition entérale doivent répondre à un profil minéral défini réglementairement. Cependant l'équilibre minéral de ces compositions est difficile à atteindre du fait des interactions entre les ingrédients.

Les protéines végétales comme les protéines de soja ont un profil minéral particulièrement intéressant. Les protéines de soja sont peu riches en calcium et en phosphate. Elles constituent également un apport en sodium, ce qui a permis à la demanderesse de limiter les apports supplémentaires en sodium. La faible teneur en phosphate des protéines de soja permet de compenser la faible teneur en calcium par un apport sous forme de phosphate de calcium dans la composition.

Selon un mode de réalisation, les protéines totales de lait sont apportées par au moins 50% en poids des protéines totales de la composition par des protéines de lait sous forme liquides et par au moins 30% en poids des protéines totales de la composition par des protéines totales de lait sous forme poudre.

Les protéines de lait sous forme liquide peuvent être apportées par du lait, du lait écrémé ou un concentré de lait écrémé. Ces protéines de lait sous forme liquide peuvent être délactosées ou non.

Avantageusement, les protéines de lait sous forme liquide sont apportées par un concentré de lait écrémé ultrafiltré et diafiltré.

La dialyse est équivalente à la diafiltration au sens de la présente demande et permet de délactoser le produit obtenu après ultrafiltration. Le terme « diafiltration » et l'adjectif « dialfiltré » seront ceux utilisés dans le reste de la demande.

Les protéines totales de lait sous forme poudre peuvent être apportées par des concentrées de protéines de lait (MPC) ou des isolats de protéines de lait (MPI).

Avantageusement, les protéines totales de lait sous forme poudre sont apportées par un isolat de protéines de lait.

L'on comprend que les protéines totales de lait sont apportées sous forme liquide et/ou solide, préférentiellement pour partie sous forme liquide et pour partie sous forme de poudre.

L'apport sous forme liquide et poudre permet d'éviter une étape d'hydratation et une étape d'évaporation dans le procédé de fabrication du produit tout en apportant une quantité importante de protéines afin d'obtenir la composition nutritionnelle à haute densité énergétique et à haute densité protéique.

Le ratio des caséines micellaires au petit-lait est proche de celle native du lait. Ce ratio en poids est d'environ 80:20 dans la source protéique laitière.

Avantageusement, les protéines végétales sont choisies parmi les protéines de soja et/ou de pois, et préférentiellement les protéines de soja.

Dans un mode de réalisation préféré, la source protéique de la composition de nutrition entérale complète consiste en des caséines micellaires en proportion majoritaire, du petit lait et des protéines de soja.

Avantageusement, la proportion entre caséines micellaires et petit-lait est proche de celle du lait, soit 80:20 en poids.

La quantité de protéines de soja n'excède pas 20% en poids des protéines totales afin de ne pas altérer les propriétés organoleptiques de la composition, et préférentiellement n'excède pas 15% en poids des protéines totales.

Le rapport en poids de caséines micellaires au petit lait et aux protéines végétales dans la composition de nutrition entérale complète est compris entre 70:15:15 et 80:10:10.

Selon un mode de réalisation, la densité énergétique de la composition est d'au moins 10 kJ/mL soit 2,4 kcal/mL.

Avantageusement, la composition de nutrition entérale complète comprend en outre au moins 8 g de lipides par 100 mL de composition, lesdits lipides apportant au moins 30% de l'apport énergétique total.

Les lipides sont majoritairement apportés sous forme d'acides gras insaturés puisque globalement meilleur pour la santé que les acides gras saturés.

Avantageusement, la composition de nutrition entérale complète comprend en outre au moins 25 g de glucides par 100 mL de composition, lesdits glucides apportant au moins 45% de l'apport énergétique total.

La composition selon l'invention ne comprend pas de fibres.

Avantageusement, le pH de la composition de nutrition entérale complète est compris entre 6,5 et 7,5.

Avantageusement, l'apport de citrate de sodium dans la composition selon l'invention est inférieur ou égal à 0,3 mg / 100 mL de composition et l'apport de citrate de potassium est inférieur ou égal à 0,3 mg / 100 mL de composition.

L'apport minéral par les citrates de potassium et de sodium limité à 0,3 mg/100mL de composition permet de limiter l'impact sur la viscosité de la composition finale. Les citrates permettent d'ajuster la teneur minérale de la composition, particulièrement en potassium et en sodium.

Selon un mode de réalisation, la composition de nutrition entérale complète thermostabilisée à longue conservation comporte :
- environ 9,6 g de protéines par 100 mL de composition, lesdites protéines apportant 16% de la teneur énergétique totale, lesdites protéines consistant en un mélange de protéines totales de lait et de protéines de soja, lesdites protéines totales de lait consistant en des caséines micellaires et du petit-lait, le rapport en poids des caséines micellaires au petit lait et aux protéines de soja étant d'environ 72:14:14,
- ladite composition ayant une densité énergétique de 10,08 kJ/mL,
- et une viscosité d'environ 217 mPa.s mesurée avec un viscosimètre rotationnel à 20°C à 50 s⁻¹.

La composition de nutrition entérale complète peut être utilisée comme source d'alimentation unique ou en complément d'alimentation d'une personne. La personne est un adulte dénutri, malade, âgé, en convalescence, ou malade chronique.

### Description

La présente invention sera mieux comprise à la lumière d'un exemple non limitatif de réalisation. La présente invention porte sur une boisson riche en énergie et nutritionnellement complète. Cette boisson est une composition de nutrition entérale qui peut être utilisée comme une solution de nutrition complète. Le patient n'a donc pas besoin systématiquement de complémenter son alimentation avec des compléments alimentaires.

La composition est prête à l'emploi et conditionnée dans des bouteilles adaptées de 125 mL, correspondant à une portion.

Dans l'exemple ci-dessous qui n'est pas limitatif, la composition de nutrition entérale est un supplément oral appartenant à la catégorie des aliments diététiques destinés à des fins médicales spéciales, destiné à couvrir les besoins nutritionnels en cas de dénutrition liée à la maladie. Il répond à la législation en vigueur sur les produits de nutrition médicale.

La composition de nutrition entérale se présente sous forme de boisson dans un conditionnement de type bouteille d'une contenance de 125 mL :
- présentant une densité énergétique de 10 kJ/mL (2.4 kcal/mL)
- présentant une teneur en protéines d'environ 9.6 g/100 ml soit environ 16% de l'apport énergétique total
- le pool protéique étant constitué de protéines totales de lait à hauteur de 86% en poids des protéines totales et de protéines végétales à hauteur de 14% en poids des protéines totales
   ∘ les protéines totales de lait consistant en un mélange de 72% de caséines micellaires et 14% de protéines de petit lait ; les protéines totales de lait (86% de l'apport protéique total) sont apportées sous forme d'un mélange représentant 52% de l'apport protéique total sous forme protéines liquides (concentré de lait écrémé ultrafiltré et diafiltré), et de 34% de l'apport protéique total sous forme de protéines totales de lait en poudre (isolat de protéines de lait)
   ∘ les protéines végétales consistant en des protéines de soja
- Contenant environ 28.8 g/100 ml de glucides, soit environ 48% de l'apport énergétique total, sous forme essentiellement de sirop de glucose ayant un dextrose équivalent compris entre 26 et 30
- Contenant environ 9.6 g/100 ml de lipides, soit environ 36% de l'apport énergétique total, apportés essentiellement sous forme d'huile de colza
- présentant un profil en vitamines et minéraux complet, de façon à pouvoir être utilisé comme seule source d'alimentation ou en complément de l'alimentation
- le sodium est préférentiellement apporté sous forme de phosphate de sodium
- comportant 0,15 mg/100 mL de citrate de sodium et 0,21 mg/100mL de citrate de potassium
- Date Limite d'Utilisation Optimale de 12 mois

La composition dans l'exemple est aromatisée à la vanille. Bien entendu, elle peut être aromatisée à n'importe quel autre parfum comme chocolat, café, fraise, framboise, citron...

La table 1 ci-dessous récapitule l'exemple de réalisation. %AET se comprend comme le pourcentage de l'apport énergétique total.
La viscosité de la composition est de 217 mPa.s mesurée à 20°C à 50 s⁻¹.
Le pH de cette composition est de 6,8.
La composition nutritionnelle est ici stérilisée UHT (Ultra Haute Température) permettant d'avoir une valeur stérilisatrice de 15 minutes.

### Préparation

La composition selon la présente invention peut être obtenue en préparant une phase aqueuse dans laquelle sont incorporés successivement en une ou plusieurs étapes le rétentat, l'eau, les glucides, les protéines, les minéraux, les vitamines, les colorants, les arômes et les éventuels autres additifs. L'eau et le rétentat peuvent être préalablement chauffés entre 30 et 60°C. La matière grasse et les émulsifiants peuvent être directement ajoutés ou préparés à part (phase grasse comportant des huiles et/ou des émulsifiants chauffée et incorporée dans la phase aqueuse).

Une phase de refroidissement et d'équilibrage est appliquée puis la composition est stérilisée avant conditionnement aseptique. Une homogénéisation est pratiquée avant ou après stérilisation.

La stérilisation est faite par chauffage selon les procédés habituels d'Ultra Haute Température.

**Table 1.profil nutritionnel d'une composition selon l'invention**

| | Unité | Valeur pour 100mL | %AET |
|---|---|---|---|
| | | | |
| Taille de la portion | ml | 125 | |
| Densité | g/l | 1140 | |
| | | | |
| Energie | kcal | 240 | |
| | kJ | 1008 | |
| Lipides (=Matières grasses) | g | 9,6 | 36,0 |
| *type de lipides* | g | | |
| acides gras saturés | g | 0,92 | |
| acides gras insaturés | g | 8,69 | |
| Glucides *(sans fibres)* | g | 28,8 | 48,0 |
| sucres (mono & di sac.) | g | 11,3 | |
| Fibres | g | 0 | 0 |
| Protéines | g | 9,6 | 16 |
| *Répartition indicative en protéines (%)* | | | |
| *Caséines micellaires* | | 72% | |
| *protéines de petit-lait* | | 14% | |
| *protéines de soja* | | 14% | |
| Sel (g) | g | 0,2525 | |
| Extrait sec | g/100ml | 49,6 | |
| Osmolalité | Osm / kg H2O | 1360 | |
| Osmolarité | Osm / L | 966 | |
| Sodium | mg/100mL | 101 | |
| Potassium | mg/100mL | 240 | |
| Calcium | mg/100mL | 168 | |
| Phophore | mg/100mL | 160 | |

## Revendications

1. Composition de nutrition entérale complète thermostabilisée à longue conservation comportant 8 à 12 g de protéines par 100 mL de composition, lesdites protéines apportant 16% de la teneur énergétique totale, lesdites protéines consistant en un mélange de protéines totales de lait et de protéines végétales, lesdites protéines totales de lait consistant en des caséines micellaires et du petit-lait, lesdites caséines micellaires représentant au moins 70% en poids des protéines totales, ladite composition ayant une densité énergétique d'au moins 8,4 kJ/mL et une viscosité comprise entre 100 et 300 mPa.s mesurée avec un viscosimètre rotationnel à 20°C à 50 s⁻¹

2. Composition de nutrition entérale complète selon la revendication 1 **caractérisée en ce que** les protéines totales de lait sont apportées par au moins 50% en poids des protéines totales de la composition par des protéines de lait sous forme liquide et par au moins 30% en poids des protéines totales de la composition par des protéines totales de lait sous forme poudre

3. Composition de nutrition entérale complète
selon l'une quelconque des revendications 1 et 2 **caractérisée en ce que** les protéines de lait sous forme liquide sont apportées par un concentré de lait écrémé ultrafiltré et diafiltré

4. Composition de nutrition entérale complète selon l'une quelconque des revendications 1 à 3 **caractérisée en ce que** les protéines totales de lait sous forme poudre sont apportées par un isolat de protéines de lait

5. Composition de nutrition entérale complète selon l'une quelconque des revendications 1 à 4 **caractérisée en ce que** les protéines végétales sont choisies parmi les protéines de soja et/ou de pois, et préférentiellement les protéines de soja

6. Composition de nutrition entérale complète selon l'une quelconque des revendications 1 à 5 **caractérisée en ce que** le rapport en poids de caséines micellaires au petit lait et aux protéines végétales est compris entre 70:15:15 et 80:10:10

7. Composition de nutrition entérale complète selon l'une quelconque des revendications précédentes **caractérisée en ce que** la densité énergétique de la composition est d'au moins 10 kJ/mL

8. Composition de nutrition entérale complète selon l'une quelconque des revendications précédentes **caractérisée en ce qu'**elle comprend en outre au moins 8 g de lipides par 100 mL de composition, lesdits lipides apportant au moins 30% de l'apport énergétique total

9. Composition de nutrition entérale complète selon l'une quelconque des revendications précédentes **caractérisée en ce qu'**elle comprend en outre au moins 25 g de glucides par 100 mL de composition, lesdits glucides apportant au moins 45% de l'apport énergétique total

10. Composition de nutrition entérale complète selon l'une quelconque des revendications précédentes **caractérisée en ce qu'**elle ne comprend pas de fibres

11. Composition de nutrition entérale complète selon l'une quelconque des revendications précédentes **caractérisée en ce que** le pH est compris entre 6,5 et 7,5.

12. Composition de nutrition entérale complète selon l'une quelconque des revendications précédentes **caractérisée en ce que** l'apport de citrate de sodium est inférieur ou égal à 0,3 mg / 100 mL de composition et **en ce que** l'apport de citrate de potassium est inférieur ou égal à 0,3 mg / 100 mL de composition

13. Composition de nutrition entérale complète thermostabilisée à longue conservation selon la revendication 1 comportant :
- 9,6 g de protéines par 100 mL de composition, lesdites protéines apportant 16% de la teneur énergétique totale, lesdites protéines consistant en un mélange de protéines totales de lait et de protéines de soja, lesdites protéines totales de lait consistant en des caséines micellaires et du petit-lait, le rapport en poids des caséines micellaires au petit lait et aux protéines de soja étant égal à 72:14:14,
- ladite composition ayant une densité énergétique de 10 kJ/mL,
- et une viscosité de 217 mPa.s mesurée avec un viscosimètre rotationnel à 20°C à 50 s⁻¹

14. Composition de nutrition entérale complète selon l'une quelconque des revendications 1 à 13 pour être utilisée comme source d'alimentation unique ou en complément d'alimentation d'une personne.

15. Composition de nutrition entérale complète selon la revendication 14 **caractérisée en ce que** la personne est un adulte dénutri, malade, âgé, en convalescence, ou malade chronique.

## Patentansprüche

1. Thermostabilisierte, vollständige, enterale Ernährungszusammensetzung mit langer Haltbarkeit, die 8 bis 12 g Protein pro 100 ml Zusammensetzung umfasst, wobei die Proteine 16 % des Gesamtenergiegehalts bereitstellen, wobei die Proteine aus einer Mischung von gesamten Milchproteinen und pflanzlichen Proteinen bestehen, wobei die gesamten Milchproteine aus mizellaren Kaseinen und Molke bestehen, wobei die mizellaren Kaseine mindestens 70 Gew.-% des gesamten Proteins ausmachen, wobei die Zusammensetzung eine Energiedichte von mindestens 8,4 kJ/ml und eine Viskosität zwischen 100 und 300 mPa·s aufweist, gemessen mit einem Rotationsviskosimeter bei 20 °C bei 50 s⁻¹.

2. Vollständige, enterale Ernährungszusammensetzung nach Anspruch 1, **dadurch gekennzeichnet, dass** die gesamten Milchproteine zu mindestens 50 Gew.-% der gesamten Proteine der Zusammensetzung durch Milchproteine in flüssiger Form und zu mindestens 30 Gew.-% der gesamten Proteine der Zusammensetzung durch gesamten Milchproteine in Pulverform bereitgestellt werden.

3. Vollständige, enterale Ernährungszusammensetzung nach einem der Ansprüche 1 und 2, **dadurch gekennzeichnet, dass** die Milchproteine in flüssiger Form durch ein Konzentrat aus ultrafiltrierter und diafiltrierter Magermilch bereitgestellt werden.

4. Vollständige, enterale Ernährungszusammensetzung nach einem der Ansprüche 1 bis 3, **dadurch gekennzeichnet, dass** die gesamten Milchproteine in Pulverform durch ein Isolat von Milchproteinen bereitgestellt werden.

5. Vollständige, enterale Ernährungszusammensetzung nach einem der Ansprüche 1 bis 4, **dadurch gekennzeichnet, dass** die pflanzlichen Proteine aus Sojabohnen- und/oder Erbsenproteinen und vorzugsweise Sojabohnenproteinen ausgewählt sind.

6. Vollständige, enterale Ernährungszusammensetzung nach einem der Ansprüche 1 bis 5, **dadurch gekennzeichnet, dass** das Gewichtsverhältnis von mizellaren Kaseinen zu Molke und zu pflanzlichen Proteinen zwischen 70:15:15 und 80:10:10 liegt.

7. Vollständige, enterale Ernährungszusammensetzung nach einem der vorhergehenden Ansprüche, **dadurch gekennzeichnet, dass** die Energiedichte der Zusammensetzung mindestens 10 kJ/ml beträgt.

8. Vollständige, enterale Ernährungszusammensetzung nach einem der vorhergehenden Ansprüche, **dadurch gekennzeichnet, dass** sie ferner mindestens 8 g Lipide pro 100 ml Zusammensetzung umfasst, wobei die Lipide mindestens 30 % der Gesamtenergiezufuhr bereitstellen.

9. Vollständige, enterale Ernährungszusammensetzung nach einem der vorhergehenden Ansprüche, **dadurch gekennzeichnet, dass** sie ferner mindestens 25 g Kohlenhydrate pro 100 ml Zusammensetzung umfasst, wobei die Kohlenhydrate mindestens 45 % der Gesamtenergiezufuhr bereitstellen.

10. Vollständige, enterale Ernährungszusammensetzung nach einem der vorhergehenden Ansprüche, **dadurch gekennzeichnet, dass** sie keine Fasern umfasst.

11. Vollständige, enterale Ernährungszusammensetzung nach einem der vorhergehenden Ansprüche, **dadurch gekennzeichnet, dass** der pH-Wert zwischen 6,5 und 7,5 liegt.

12. Vollständige, enterale Ernährungszusammensetzung nach einem der vorhergehenden Ansprüche, **dadurch gekennzeichnet, dass** die Zufuhr von Natriumcitrat kleiner oder gleich 0,3 mg/100 ml der Zusammensetzung ist und dass die Zufuhr von Kaliumcitrat kleiner oder gleich 0,3 mg/100 ml der Zusammensetzung ist.

13. Thermostabilisierte, vollständige, enterale Ernährungszusammensetzung mit langer Haltbarkeit nach Anspruch 1, umfassend:
- 9,6 g Protein pro 100 ml Zusammensetzung, wobei die Proteine 16 % des Gesamtenergiegehalts bereitstellen, wobei die Proteine aus einer Mischung von Gesamtmilchproteinen und Sojaproteinen bestehen, wobei die Gesamtmilchproteine aus mizellaren Kaseinen und Molke bestehen, wobei das Gewichtsverhältnis von mizellaren Kaseinen zu Molke und Sojaproteinen gleich 72:14:14 ist,
- wobei die Zusammensetzung eine Energiedichte von 10 kJ/ml
- und eine Viskosität von 217 mPa s aufweist, gemessen mit einem Rotationsviskosimeter bei 20 °C bei 50 s⁻¹.

14. Vollständige, enterale Ernährungszusammensetzung nach einem der Ansprüche 1 bis 13 zur Verwendung als einzige Nahrungsquelle oder als Nahrungsergänzung einer Person.

15. Vollständige, enterale Ernährungszusammensetzung nach Anspruch 14, **dadurch gekennzeichnet, dass** die Person ein unterernährter, kranker, älterer, genesender oder chronisch kranker Erwachsener ist.

## Claims

1. Thermostabilized, long-life complete enteral nutritional composition, comprising 8 to 12 g of protein per 100 mL of composition, said protein providing 16% of the total energy content, said protein consisting of a mixture of complete milk proteins and vegetable proteins, said complete milk proteins consisting of micellar caseins and whey, said micellar caseins representing at least 70% by weight of the total protein, and said composition having an energy density of at least 8.4 kJ/mL and a viscosity of between 100 and 300 mPa.s measured using a rotational viscometer at 20°C at 50 s⁻¹.

2. Complete enteral nutritional composition according to claim 1, **characterized in that** the complete milk proteins are provided by at least 50% by weight of the total protein in the composition by milk proteins in liquid form, and by at least 30% by weight of the total protein in the composition by complete milk proteins in powder form.

3. Complete enteral nutritional composition according to either claim 1 or claim 2, **characterized in that** the milk proteins in liquid form are provided by a concentrate of ultrafiltered and diafiltered skim milk.

4. Complete enteral nutritional composition according to any of claims 1 to 3, **characterized in that** the complete milk proteins in powder form are provided by a milk protein isolate.

5. Complete enteral nutritional composition according to any of claims 1 to 4, **characterized in that** the vegetable proteins are selected from among soy and/or pea proteins, and are preferably soy proteins.

6. Complete enteral nutritional composition according to any of claims 1 to 5, **characterized in that** the weight ratio of micellar caseins to whey and vegetable proteins is between 70:15:15 and 80:10:10.

7. Complete enteral nutritional composition according to any of the preceding claims, **characterized in that** the energy density of the composition is at least 10 kJ/mL.

8. Complete enteral nutritional composition according to any of the preceding claims, **characterized in that** it further comprises at least 8 g of lipids per 100 mL of composition, said lipids providing at least 30% of the total energy amount.

9. Complete enteral nutritional composition according to any of the preceding claims, **characterized in that** it further comprises at least 25 g of carbohydrates per 100 mL of composition, said carbohydrates providing at least 45% of the total energy amount.

10. Complete enteral nutritional composition according to any of the preceding claims, **characterized in that** it does not comprise fiber.

11. Complete enteral nutritional composition according to any of the preceding claims, **characterized in that** the pH is between 6.5 and 7.5.

12. Complete enteral nutritional composition according to any of the preceding claims, **characterized in that** the amount of sodium citrate is less than or equal to 0.3 mg/100 mL of composition, and **in that** the amount of potassium citrate is less than or equal to 0.3 mg/100 mL of composition.

13. Thermostabilized, long-life complete enteral nutritional composition according to claim 1, comprising:
- 9.6 g of protein per 100 mL of composition, said protein providing 16% of the total energy content, said protein consisting of a mixture of complete milk proteins and soy proteins, said complete milk proteins consisting of micellar caseins and whey, the weight ratio of micellar caseins to whey and soy proteins being equal to 72:14:14,
- said composition having an energy density of 10 kJ/mL,
- and a viscosity of 217 mPa.s measured using a rotational viscometer at 20°C at 50 s⁻¹.

14. Complete enteral nutritional composition according to any of claims 1 to 13, for use as a sole food source or as a supplement to a person's diet.

15. Complete enteral nutritional composition according to claim 14, **characterized in that** the person is a malnourished, ill, elderly, convalescent or chronically ill adult.
